# EUROPÄISCHE PATENTANMELDUNG

(11) **EP 2 181 674 A1**
(43) Veröffentlichungstag der Anmeldung: **05.05.2010**
(21) Anmeldenummer: 09012675.6
(22) Anmeldetag: 07.10.2009
(51) Int. Cl.: A61F 2/44

(54) **Justierbare Bandscheibenprothese**

(30) Priorität: 14.10.2008 DE 102008051588
(71) Anmelder: Global Medical Consulting Gmbh, 94327 Bogen (DE)
(72) Erfinder: Bertagnoli, Rudolf, Dr., 94315 Straubing (DE)

(57) **Zusammenfassung**

Es wird eine bewegliche Bandscheibenprothese mit einem Drehpunkt vorgeschlagen, der vor, während und/oder nach der Implantation gezielt verlagert werden kann. Der Drehpunkt wird im implantiertem Zustand der Prothese durch die dreidimensionalen Bewegungsachsen eines Wirbelsegments definiert.

## Beschreibung

Die Erfindung bezieht sich auf eine bewegliche Bandscheibenprothese mit einem Drehpunkt, bei dem sich im implantierten Zustand dreidimensionale Bewegungsachsen eines Wirbelsegments kreuzen.

Die Implantation von beweglichen Bandscheibenprothesen bedarf einer großen Sorgfalt, was den Drehpunkt anbelangt. Der natürliche Drehpunkt, über den ein gesamtes Wirbelsegment bewegt wird, liegt normalerweise im hinteren Drittel der Bandscheibe. Darüber hinaus bewegt sich der natürliche Drehpunkt auf einer Ebene etwa einem Kreis folgend, je nach den Positionen oder Stellungen der Wirbelkörper des Patienten.

Konventionelle bewegliche Bandscheibenprothesen definieren einen Drehpunkt, der im implantierten Zustand eine fixe Position einnimmt. Wenn dieser nicht in der Gleichgewichtsebene des natürlichen Bewegungsablaufes liegt, kann es zum vorzeitigen Verschleiß der Prothese und ggf. auch zum weiteren Verschleiß des Bewegungssegments führen.

Gemäß der DE 103 61 772 A1 soll diesem Nachteil mit einer Bandscheibenprothese dadurch Rechnung getragen werden, daß zwischen zwei mit den angrenzenden Wirbelkörpern verbundenen künstlichen Endplatten eine schwimmend gelagerte, halbkugelförmige Zwischenwirbelscheibe vorgesehen ist, die zwischen den künstlichen Endplatten Translationsbewegungen durchführen kann. Hierbei wird der Drehmittelpunkt der Rotations- und Beugebewegungen zwar mit der Bewegung des Rückgrades verlagert, jedoch erfolgt dieses unkontrolliert, die Prothese schlingert unnatürlich und schädigt dabei die übrigen Gelenke.

Der Erfindung liegt die Aufgabe zugrunde, eine Prothese der eingangs genannte Art zu entwickeln, mit der die Implantation im Hinblick auf eine möglichst genaue Platzierung des Drehpunktes vereinfacht werden kann.

Die Aufgabe wird erfindungsgemäß mit den Merkmale des Anspruchs 1 gelöst. Demnach ist die Lage zumindest der den Drehpunkt definierenden Komponente der Bandscheibenprothese translatorisch verstellbar oder verschiebbar ausgestaltet.

Durch diese Möglichkeit einer Justierung bzw. Nachjustierung der Prothese kann die Lage des Drehpunktes gezielt platziert bzw. korrigiert und damit weitgehend optimal gelagert werden, ohne die Lage der Prothese und den zwangsweise Verankerungszustand zu verändern. Die Anwendung findet an konventionellen und erprobten Prothesensystemen statt.

Die Erfindung hat den Vorteil, daß die Prothese zur Optimierung der Lage des Drehpunktes justiert wird, wobei die den Drehpunkt definierenden Prothesenkomponenten oder -modulen nicht unkontrolliert schlingern, wie es bei dem erwähnten Stand der Technik der Fall ist.

In der Regel werden modulare Prothesen verwendet, bei denen die den Drehpunkt definierenden Prothesenmodule relativ zu den natürlichen Endplatten der angrenzenden Wirbelkörper translatorisch ein- oder zweidimensional verschiebbar ausgestaltet werden. Auf die Weise kann der Drehpunkt weitgehend austariert werden, derart, daß das gesamte Bewegungssegment des Rückgrads stabil bzw. im Gleichgewicht gehalten werden kann. Die Einstellung des Drehpunktes kann nach Kenntnis der Anatomie des Patienten vor der Implantation erfolgen. Es ist sinnvoll, eine Ausgestaltung der Prothese zu wählen, die eine Einstellung der Drehpunktlage auch während der Operation erlaubt, was auch die Möglichkeit eröffnet, Korrekturen nach erfolgter Operation oder in einem Revisionseingriff vorzunehmen.

Die Komponenten einer modularen Prothese werden relativ zueinander beweglich bzw. verschiebbar ausgestaltet. Durch Normierung der Komponenten ist der Austausch bzw. die für den jeweiligen Patienten passende Kombination einfach und rasch zusammenstellbar. Dabei können Grundkomponenten, wie künstliche Endplatten, einheitlich ausgebildet werden.

Eine vorteilhafte Prothese besteht aus zwei künstlichen Endplatten, die zwischen sich ein um den Drehpunkt bewegliches Zwischenmodul aufnehmen, und daß das Zwischenmodul parallel zu den Endplattenebenen verschiebbar ausgestaltet ist.

Das bewegliche Zwischenmodul besteht gemäß einer fertigungstechnisch einfachen Lösung aus zwei Komponenten, z.B. einem Halbkugel-Pfannen Paar. Es werden entweder beide Komponenten als Einheit verschiebbar ausgestaltet oder gemäß einer einfachen Lösung ist nur eines der beiden Komponenten relativ zur zugehörigen Endplatte verschiebbar gestaltet, während die andere Komponente schwimmend mit der zweiten Endplatte gelagert ist, derart, dass sie bei tanslatorischen Verschiebunge der ersten Komponente mitgezogen bzw. verschoben wird.

Ein fertigungstechnisch einfacher Verstellmechanismus besteht in einer Schraubverstellung, bei der das Zwischenmodul oder nur eines dessen Komponenten eindimensional gegenüber Referenzmodulen, z.B. künstlichen Endplatten verschoben werden kann. Zum Beispiel wird diese Verstellbarkeit in Richtung Ventral-Dorsal des Patienten gewählt, wenn die optimale seitliche Position des Drehpunktes durch symmetrische Ausgestaltung der Prothese erreichbar ist. Diese Lösung erlaubt im Falle einer Fehlstellung, die sich nach der Operation herausstellt, eine Nachjustierung durch Betätigung der Stellschraube, was mit geringem nachoperativen Aufwand durchführbar ist.

Vorzugsweise wird eine zweidimensionale Translation vorgesehen, die es ermöglicht, den Drehpunkt der Prothese sowohl in Richtung ventral/dorsal als auch seitlich optimal auf die Drehpunktposition des natürlichen Bewegungssegmentes einzustellen.

Translatorische Verstellmöglichkeiten mittels Stellschrauben können manuell oder über Induktionspulen elektrisch betätigt werden. Letztere Fall ermöglicht Korrekturen auch von außen, d.h. ohne operativen Eingriff.

Es sind alle technisch möglichen Lösungen zur Verschiebung von translatorisch beweglichen Prothesenmodulen anwendbar, die sich dimensionsmäßig für Bandscheibenimplantaten realisieren lassen.

Ein natürliches Wirbelsegment übt neben den Rotationsbewegungen auch translatorische Bewegungen denkrecht zur Hochachse aus. Der Drehpunkt des natürlichen Bewegungsapparates bewegt sich bei unterschiedlichen Wirbelkörperpositionen auf einer Ebene annähernd einem Kreis folgend. Um diese natürliche Bewegung des Drehpunktes nachzubilden, wird gemäß einer weiteren Ausgestaltung der Erfindung ein Stellmotor vorgeschlagen, mit dem die translatorische Bewegungen des Drehpunktes entsprechend der natürlichen Bewegung nachgeahmt werden kann. Bewegungssensoren an Wirbelkörpern und/oder Muskeln können dem entsprechend programmierten Stellmotor die notwendigen Signale liefern.

Die technische Realisierung von Lösungen zur translatorischen Verschiebung von Modulkomponenten einer Bandscheibenprothese ist in der einschlägigen Technik bzw. in den einschlägigen Fachwelt zu suchen. Wesentlich hier ist der Gedanke, der möglichst genauen Einstellung des künstlichen Drehpunktes durch einmalige, oder nachgeholte oder kontinuierliche translatorische Verschiebung von Prothesenmodulen.

Die Erfindung wird anhand von in der Zeichnung schematisch dargestellten Ausführungsbeispielen näher beschrieben. Es zeigen:
Fig. 1 ein Wirbelsegment,
Fig. 2a und b ein Ausführungsbeispiel im Schnitt und
Fig. 3 eine Verstellmechanik.

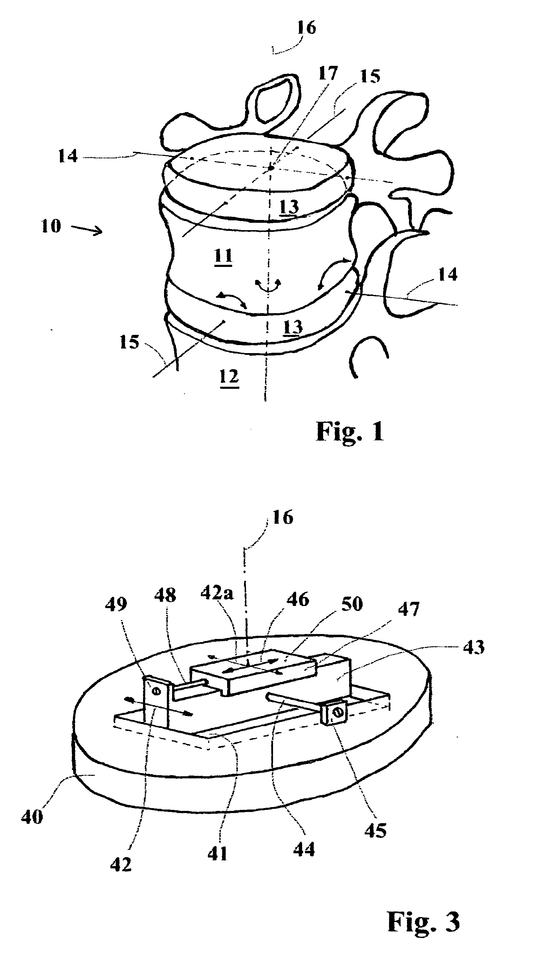

In Fig. 1 ist ein Wirbelsegment 10 dargestellt, das aus zwei Wirbelkörper 11,12 und einer Bandscheibe 13 besteht. Bei Bewegungen des Patienten üben die Wirbelkörper 11,12 des Wirbelsegments 10 Relativbewegungen aus, bei denen sie um eine Extension/Beugungsachse 14, Seitenneigungsachse 15 sowie Drehung um eine Hochachse 16 ausüben. Diese Achsen 14-16 schneiden sich im Drehpunkt 17. Dieser natürliche Drehpunkt 17 kommt in etwa im hinteren drittel der Bandscheibe zu liegen.

Wird die natürliche Bandscheibe 13 durch ein bewegliches Implantat ersetzt, so wird ein künstlicher Drehpunkt zur Erhaltung der Beweglichkeit durch entsprechend beweglich ausgebildete Implantatkomponenten bzw. -modulen nachgebildet.

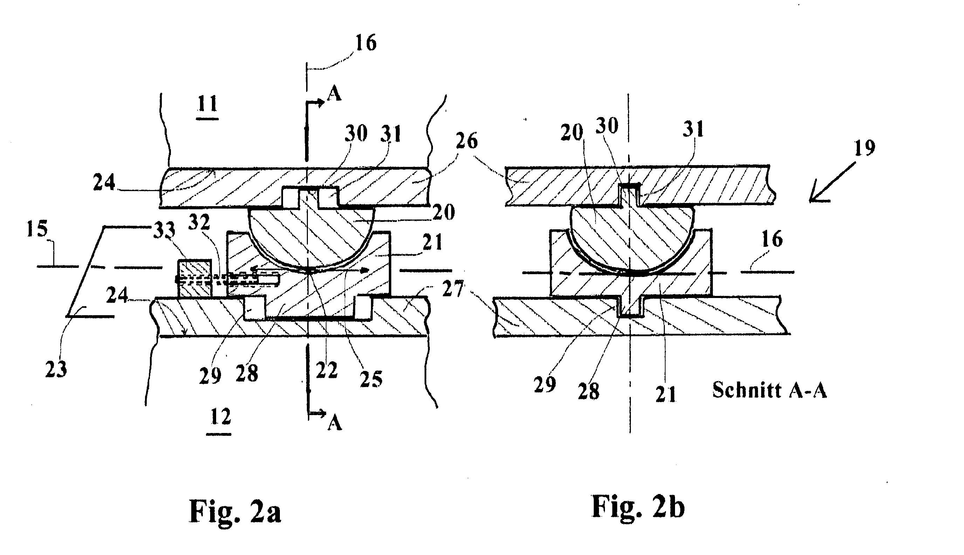

Ein einfaches Beispiel ist eine Halbkugel/Pfannen-Prothese, wie sie in Fig. 2a im Längsschnitt in Richtung Ventral-Dorsal dargestellt ist. Fig. 2b zeigt einen Längsschnitt A-A quer zur Fig. 2a.

Die Halbkugel 20 hat innerhalb der Pfanne 21 eine dreidimensionale Beweglichkeit, mit einem künstlichen Drehpunkt 22.

Die richtige Platzierung des künstlichen Drehpunktes 22 auf einer Ebene 23 parallel zu den natürlichen Endplatten 24 der Wirbelkörper 11,12 spielt für die Stabilität der Wirbelsäule eine wichtige Rolle.

Gemäß dem Ausführungsbeispiel nach Fig. 2a/2b ist eine Justierungsmöglichkeit vorgesehen, mit der die Lage des künstlichen Drehpunkts 22 in Ventral-Dorsal-Richtung 25 translatorisch verschoben werden kann. Die modular ausgebildete Prothese 19 besteht aus zwei künstlichen Endplatten 26,27, die mit jeweils einem Wirbelkörper 11, bzw. 12 fest verbunden werden und die die Halbkugel 20 bzw. die Pfanne 21 mit Spiel aufnehmen.

Die Pfanne 21 hat einen länglichen, ventral-dorsal gerichteten Steg 28, der in eine längliche Vertiefung 29 der unteren künstlichen Endplatte 27 hineinragt, derart, daß der Steg 28 in der Vertiefung 29 längsverschiebbar ist. In ähnlicher Weise ragt ein Steg 30 der Halbkugel 20 in eine entsprechende Vertiefung 31 in der oberen künstlichen Endplatte 26 hinein, derart, daß Halbkugel 20 und Pfanne 21 gemeinsam und damit der Drehpunkt 22 in ventral-dorsal Richtung bewegt werden können.

Um die Lage des Drehpunktes 22 zu justieren ist eine Schraube 32 drehbeweglich in einen Stutzen 33 gelagert, der an der unteren künstlichen Endplatte 27 fixiert ist. Die Schraube 32 ragt in eine Gewindebohrung 34 der Pfanne 21, derart, daß beim Drehen der Schraube 32 die Pfanne 21 und damit der Steg 28 innerhalb der Vertiefung 29 hin und her bewegt werden können. Die schwimmend gehaltene Halbkugel 20 wird mit der Pfanne 21 mitgezogen, so daß der Drehpunkt 22 durch Justierung an der Schraube 32 in die gewünschte Ventral-Dorsallage gebracht werden kann.

Die Justierung kann nach Kenntnis der genauen Anatomie der Operationsstelle des Patienten vor der Implantation der Bandscheibenprothese 19 erfolgen. In der Regel wird die genaue Justierung der Lage des Drehpunktes 22 während der Implantation vorgenommen. Bei dem beschriebenen Ausführungsbeispiel ist auch eine Justierung zu einem späteren Zeitpunkt, wenn sich z.B. ein Ungleichgewicht im Wirbelsegment herausstellt, möglich. Hierzu ist lediglich ein minimal-invasiver Eingriff zum Drehen der Schraube 32 erforderlich.

Die Bandscheibenprothese 19 kann in modularer, vorzugsweise normierter modularer Bauweise hergestellt werden, wobei die mit den Vertiefungen 29,31 ausgestatteten künstlichen Endplatten 26 und 27 auf Dauer an den angrenzenden Wirbelkörpern 11,12 fixier werden. Austauschbar ist das bewegliche Zwischenmodul.

Der Übersicht halber ist eine Bandscheibenprothese mit translatorischer Verschiebbarkeit des Drehpunktes 22 nur in einer Richtung 25 beschrieben.

Die Erfindung erstreckt sich aber auf alle möglichen Konstruktionen und Mechanismen zur Verschiebung des Drehpunktes in einer oder zwei Richtungen oder auf einer Ebene.

Ebenfall der Übersicht halber wird eine in zwei Richtungen verschiebbare Mechanik in Fig. 3 als Prinzipzeichnung dargestellt.

Als Referenzebene dient eine Platte 40, die eine rechteckige Vertiefung 41 aufweist, in der ein beweglicher, in einer ersten Richtung 42 beweglicher Steg 43 platziert ist. Die translatorische Bewegung in dieser ersten Richtung 42 wird mittels einer Schraube 44 bewirkt, die sich an einem Stutzen 45 abstützt, der mit der Referenzebene 40 eine Einheit bildet.

Der in der ersten Richtung 42 bewegliche Steg 43 bildet die Referenzstütze für die zweite Bewegungsrichtung 46 und nimmt einen Schlitten 47 auf, der entlang des Steges 43 verschiebbar aufsitzt. Der Schlitten 47 wird mittels einer zweiten Schraube 48 bewegt, die ihrerseits sich an einem mit dem Steg 43 einheitlich verbundenen Stutzen 49 abstützt.

Der Schlitten 47 kann somit mit dem Steg 43 in der ersten Richtung 42a hin und her geschoben werden, während er auf dem Steg 43 in der zweiten, Querrichtung 46 hin und her bewegbar ist.

Auf eine Bandscheibenprothese angewendet, wird die Referenzplatte 40 eine künstliche Endplatte darstellen und der Schlitten 47 könnte als Pfanne ausgestaltet sein. Damit wird der Drehpunkt 22 des Beispieles gemäß Fig. 2a innerhalb einer rechteckigen Ebene translatorisch verschiebbar sein, so daß über die beiden Verstellschrauben 44 und 48 der Drehpunkt 22 im implantierten Zustand der Bandscheibenprothese genau platziert werden kann.

Es sind Techniken denkbar, die eine elektronische oder elektroinduktive Steuerung z.B. der Schrauben ermöglichen, wodurch eine Nachjustierung des Drehpunktes ohne direkten operativen Eingriff durchgeführt werden kann. Die moderne Elektronik macht auch Steuergeräte möglich, die eine Verschiebung des Drehpunktes während der Bewegung des Patienten vollzieht. Der künstliche Drehpunkt 22, der nach dem Beispiel von Fig.3 sich auf einer rechteckigen Ebene bewegen kann, wird durch Auslegung der Bewegungstechnik die natürliche Kreisfläche abdecken können. Das hat den Vorteil, daß bei entsprechender Elektrotechnik eine automatische kontinuierliche Verschiebung der Drehpunktlage in Anhängigkeit der Stellung des Rückgrads und der Bewegung des Patienten durchführbar ist.

## Patentansprüche

1. Bewegliche Bandscheibenprothese mit einem Drehpunkt, bei dem sich im implantierten Zustand die dreidimensionalen Bewegungsachsen eines Wirbelsegments kreuzen, **dadurch gekennzeichnet, daß** die Prothese (19) derart ausgestaltet ist, daß vor, während und/oder nach der Implantation eine gezielte Verlagerung (25;42a,46) des Drehpunkts (22) durchführbar ist.

2. Bandscheibenprothese nach Anspruch 1, **dadurch gekennzeichnet, dass** die Prothese (19) mit Mitteln (32;44,48) ausgestattet ist, die eine Lagejustierung (25;42a,46) des Drehpunkts (22) gegenüber festen Bauteilen (26,27;40) erlaubt.

3. Bandscheibenprothese nach Anspruch 2, **dadurch gekennzeichnet dass** als Mittel Stellschrauben (32;44,48) für die Lagejustierung (25;42a,46) des Drehpunkts (22) vorgesehen sind.

4. Bandscheibenprothese nach einem der vorhergehenden Ansprüche, **dadurch gekennzeichnet, dass** die Prothese (19) Induktionsspulen aufweist, über die die Drehpunktposition von außen steuerbar ist.

5. Bandscheibenprothese nach Anspruch 1 oder 2, **dadurch gekennzeichnet, dass** der Prothese (19) ein Stellmotor zugeordnet ist, mit dem die Position des Drehpunktes (22) kontinuierlich an die Gegebenheiten im menschlichen Körper angepaßt werden kann.

6. Bandscheibenprothese nach einem der vorhergehenden Ansprüche, **dadurch gekennzeichnet, dass** eine modulare Prothese (19) vorgesehen ist, die aus mit den Wirbelkörpern fest verankerbaren Modulkomponenten (26,27) sowie relativ zu diesen translatorisch verschiebbaren beweglichen und ggf. austauschbaren Modulkomponenten (20,21;47) besteht.

7. Bandscheibenprothese nach Anspruch 6, **dadurch gekennzeichnet, dass** als fest verankerbare Modulkomponenten zwei künstliche Endplatten (26,27) vorgesehen sind, die zwischen sich ein annähernd parallel zu den Endplatten verschiebbares Zwischenmodul (20,21) aufnehmen.

8. Bandscheibenprothese nach Anspruch 7, **dadurch gekennzeichnet, dass** das bewegliche Zwischenmodul (20,21) aus mindestens zwei Komponenten besteht, wobei eine erste Komponente (21) relativ zur zugehörigen künstlichen Endplatte (27) verschiebbar befestigt ist, während eine zweite Komponente (20) wenigstens zum Teil schwimmend zwischen der zweiten Endplatte (26) und der ersten Komponente (21) gelagert ist.

9. Bandscheibenprothese nach Anspruch 8, **dadurch gekennzeichnet, dass** das bewegliche Zwischenmodul (20,21) austauschbar ausgestaltet ist.
